# EUROPÄISCHE PATENTANMELDUNG

(11) **EP 1 022 564 A2**
(43) Veröffentlichungstag der Anmeldung: **26.07.2000**
(21) Anmeldenummer: 00101172.5
(22) Anmeldetag: 21.01.2000
(51) Int. Cl.: G01N 31/12, G01N 33/18

(54) **Verbrennungsofen für die Verbrennung von flüssigen Proben**

(30) Priorität: 21.01.1999 DE 19902277
(71) Anmelder: LAR Analytik und Umweltmesstechnik GmbH, 10179 Berlin (DE)
(72) Erfinder: Arts, Werner, Dr., 10825 Berlin (DE)
(74) Vertreter: Heinze, Ekkehard, Dipl.-Phys. Dr.

(57) **Zusammenfassung**

Verbrennungsofen (1) für die Verbrennung von flüssigen Proben zur Bestimmung von Inhaltsstoffen, mit einer Schleuseneinrichtung (3) zum Eintrag der Probe mittels einer Probenahmeeinrichtung (15).

## Beschreibung

Die Verbrennung flüssiger Proben mit anschließendem quantitativen Nachweis der Verbrennungsprodukte stellt ein wichtiges Verfahren beispielsweise für die Analyse von Abwässern dar. Insbesondere läßt sich auf diese Weise der Kohlenstoffgehalt (TC = Total Carbon) des Abwassers und daraus schließlich auch der Gehalt an organischem Kohlenstoff (TOC = Total Organic Carbon) bestimmen.

Zur Ausführung dieser Verbrennung geeignete Verbrennungsöfen sind an sich bekannt. Ein vorteilhaftes Verfahren zur TOC-Bestimmung und ein neuartiger Verbrennungsofen sind in der DE 197 27 839 A1 der Anmelderin beschrieben.

In einen solchen Ofen wird eine kleine Probe des Abwassers oder der anderen flüssigen Probe eingespritzt, und zur Gewährleistung einer ausreichenden Genauigkeit der Meßergebnisse dürfen keine sonstigen Stoffe in den Ofen gelangen. Da das Einbringen der flüssigen Probe in den Verbrennungsofen üblicherweise mit einer Injektionsspritze erfolgt, die in einem Reservoir für die Probenflüssigkeit aufgezogen und deren Inhalt in den Verbrennungsofen gespritzt wird, weisen bekannte Verbrennungsöfen eine mit der Nadel der Injektionsspritze durchstoßbare und sich nach dem Herausziehen der Nadel grundsätzlich wieder selbst abdichtende Gummimembran, ein sogenanntes Septum, auf.

Die Haltbarkeit dieser Verbrennungsofenverschlüsse hat sich jedoch als begrenzt erwiesen, und nach einigen hundert Beschickungsvorgängen - im Routinebetrieb einer Abwasserreinigungsanlage also schon nach einigen Tagen Betriebsdauer - treten Undichtigkeiten auf, die eine Auswechslung des Ofenverschlusses erfordern. Zudem hat sich gezeigt, daß mitunter beim Durchstechen des Septums mit der Injektionsspritze Partikel desselben zusammen mit der Probenflüssigkeit in den Innenraum des Verbrennungsofens gelangen, was zu dramatisch verfälschten Nachweisergebnissen führt.

Der Erfindung liegt daher die Aufgabe zugrunde, einen Verbrennungsofen mit einem haltbaren und die Gefahr einer Verunreinigung des Verbrennungsraums wesentlichen verringernden Verschluß anzugeben.

Diese Aufgabe wird durch einen Verbrennungsofen mit den Merkmalen des Anspruchs 1 gelöst.

Die Erfindung schließt die technische Lehre ein, einen nach dem Schleusenprinzip arbeitenden Verschluß zur Beschickung mit der Probe vorzusehen.

In einer vorteilhaften Ausführung umfaßt die Schleuseneinrichtung auf der Verbrennungsofen-Außenseite einen Öffnungsbereich, der in Abstimmung auf die im Beschickungssystem vorgesehene Probenahmeeinrichtung so ausgebildet ist, daß er durch Einführung der Probenahmeeinrichtung - in der Regel der Nadel einer Injektionsspritze - dicht verschließbar ist. Weiterhin umfaßt sie an der Innenseite des Verbrennungsofens bzw. zum Verbrennungsraum hin einen steuerbaren Verschluß. Dieser wird verschlossen gehalten, solange in die Beschickungsöffnung keine Probenahmeeinrichtung eingreift, und er wird geöffnet, wenn eine Probenahmeeinrichtung dort eingesetzt ist und der Verbrennungsraum mit der Probe beschickt werden soll. Auf diese Weise wird sichergestellt, daß der Verbrennungsofen sowohl außerhalb von Beschickungsvorgängen als auch während solcher Vorgänge gegenüber der Umgebung abgeschlossen und nur während des Beschickungvorgangs für die Probenahmeeinrichtung geöffnet ist.

In Anpassung an die üblicherweise zur Probenahme und Beschickung von Verbrennungsöfen verwendeten Injektionsspritzen ist die Beschickungsöffnung als im Querschnitt kreisförmige Öffnung eines - zum Ausgleich von geringfügigen Positionsfehlern bei der Beschickung bevorzugt elastischen oder elastisch gelagerten - Führungsrings ausgebildet.

Der innere Verschluß umfaßt insbesondere ein manuell oder - speziell in einem automatisierten Probenahme- und Beschickungssystem - in Abhängigkeit vom Verschlußzustand der Beschickungsöffnung automatisch gesteuertes Absperrorgan, insbesondere ein Absperrventil oder einen Absperrhahn oder Absperrschieber. Dessen Öffnungsquerschnitt ist speziell an den Außenquerschnitt des in die Beschickungsöffnung eingreifenden Abschnitts der Probenahmeeinrichtung, speziell den Außenquerschnitt der Nadel einer Probenahmespritze, angepaßt, so daß dieser durch das Absperrorgan in den Verbrennungsofen eingeschoben werden kann. Abgesehen von einer möglichst spielfreien Anpassung des Öffnungsquerschnitts an den Nadelquerschnitt ist auch hier im Interesse eines möglichst dichten Abschlusses gegen den Außenraum eine gewisse Elastizität bzw. ggfs. das Vorsehen von Dichtmitteln am Absperrorgan günstig.

Eine besonders rationelle Beschickung des Verbrennungsofens, die die sich ständig wiederholende Ausführung von Routinebestimmungen, beispielsweise von Abwasserinhaltsstoffen, erheblich erleichtert, wird durch eine auf den vorgeschlagenen Verbrennungsofen angepaßte Gesamtanordnung zur Entnahme einer flüssigen Probe aus einem Reservoir (Behälter oder Fluidleitung) und deren Überführung in den Verbrennungsofen möglich.

Zu dieser Gesamtanordnung gehören insbesondere eine Verfahreinrichtung zum Transport der Probenahmeeinrichtung zwischen einer ersten Stellung, in der sie in das Reservoir eingreifen kann, und einer zweiten Stellung, in der sie in den Verbrennungsofen eingreifen kann. Die Verfahreinrichtung umfaßt insbesondere einen elektromotorischen Antrieb, der durch eine Koordinatensteuereinrichtung angesteuert wird. Letztere ist für das Zusammenwirken mit der bevorzugten Ausführung des Verbrennungsofens so programmiert, daß die Einführung des Eingriffsabschnitts der Probenahmeeinrichtung in den Verbrennungsofen in zwei Schritten erfolgt, die insbesondere in ein und derselben Vorschubrichtung ausgeführt werden. Sie ist dann weiterhin so ausgebildet, daß nach Ausführung des ersten Schritts ein Öffnungssteuersignal an den Antrieb der Schleuseneinrichtung ausgegeben wird. Zur Vermeidung von Beschädigungen der Probenahmeeinrichtung (Injektionsspritze) und des Ofenverschlusses ist - anders als bei den bekannten Septum-Ofenverschlüssen - eine präzise Ausrichtung der Probenahmeeinrichtung mit der Beschickungsöffnung und dem Öffnungsbereich des Absperrorgans (im geöffneten Zustand) und auch die Vorgabe von Vorschublängen für den ersten und zweiten Schritt in Abstimmung auf die Schleusenabmessungen erforderlich.

Zweckmäßigkeiten und Vorteile der Erfindung ergeben sich im übrigen aus den Unteransprüchen sowie der nachfolgenden Beschreibung einer bevorzugten Ausführungsform unter Bezugnahme auf die Figuren. Von diesen zeigen:
- Fig. 1a bis 1c: den Verschlußabschnitt eines Verbrennungsofens in einer Ausführungsform zusammen mit einer als Injektionsspritze ausgeführten Probenahmeeinrichtung in drei Phasen des Beschickungvorgangs in schematischer Darstellung und
- Fig. 2: ein Funktions-Blockschaltbild einer Ausführungsform einer Gesamt-Beschickungsanordnung.

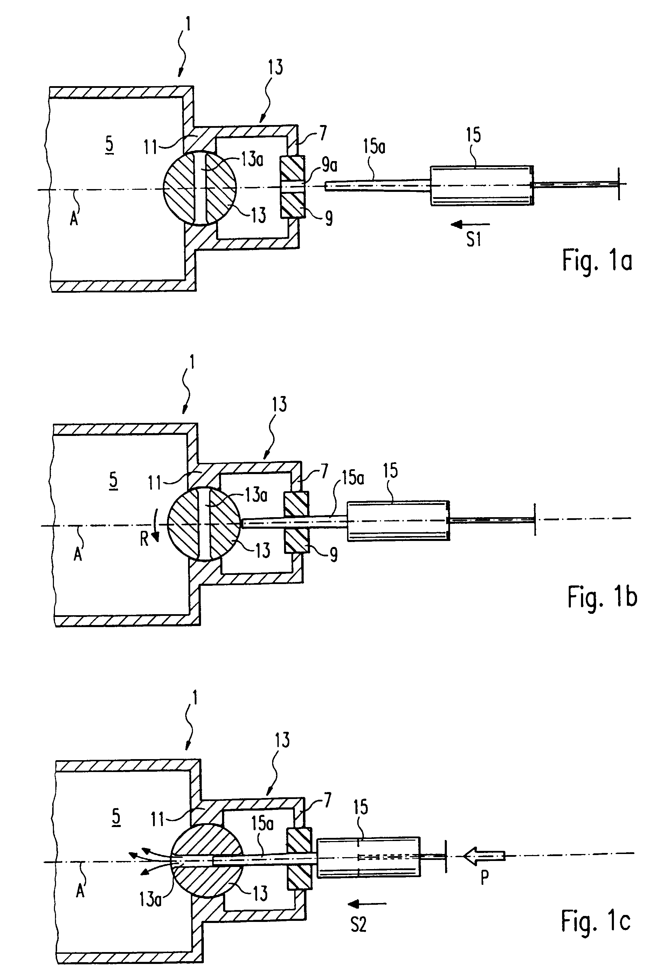

Wie in Fig. 1a bis 1c dargestellt, weist ein Verbrennungsofen 1 eine Schleuseneinrichtung 3 auf, die einen Verbrennungsraum 5 zur Umgebung hin abschließt. Die Schleuseneinrichtung 3 umfaßt einen in einen ersten, äußeren Wandungsabschnitt 7 eingesetzten Kunststoff-Führungsring 9 (beispielsweise aus Teflon), und ein in einem zweiten, inneren Wandungsabschnitt 11 angeordnetes Kugelventil 13. Das Kugelventil 13 ist in Fig. 1a in Schließstellung gezeigt und verschließt den Ofeninnenraum 5 dicht gegenüber der Umgebung. Ein Fluidkanal 13a des Kugelventils 13 sowie eine - im Querschnitt ebenfalls kreisförmige - zentrische Öffnung 9a im Führungsring 9 sind in ihrem Querschnitt auf den Außenquerschnitt einer Nadel 15a einer Probenahmespritze 15 angepaßt. Der Abstand zwischen der Öffnung 9a und dem Fluidkanal 13a der Schleuseneinrichtung 3 ist so gewählt, daß die Nadel 15a der Injektionsspritze im Eingriffszustand (siehe Fig. 1c) in beide zugleich eingreift. In Fig. 1a ist die Injektionsspritze jedoch im Bereitschaftszustand für die Beschickung des Verbrennungsofens, vor der Öffnung 9a des Führungsrings 9 und mit dieser längs einer gemeinsamen Achse A ausgerichtet, dargestellt. In einem ersten Vorschubschritt S1 wird die Injektionsspritze aus diesem Ausgangszustand zunächst so weit längs der Achse A vorgeschoben, daß die Nadel 15a in die Öffnung 9a eingreift und diese dicht abschließt.

Damit ist der in Fig. 1b gezeigte Zustand erreicht, in dem durch Drehen des Kugelventils 13 um 90° (in der Figur durch den mit R bezeichneten Pfeil gekennzeichnet) um eine senkrecht auf der Zeichenebene stehende Achse das Kugelventil geöffnet wird.

Sobald das Kugelventil 13 geöffnet ist, wird in einem zweiten Vorschubschritt S2 die Probenahmespritze 15 längs der Achse A um einen weiteren vorbestimmten Vorschubbetrag derart vorgeschoben, daß die Nadel 15a in den Fluidkanal 13a des Kugelventils 13 eingreift. Anschließend wird durch axialen Druck P auf den (nicht gesondert bezeichneten) Kolben der Injektionsspritze 15 die in dieser aufgenommene Probenflüssigkeit in den Verbrennungsraum 5 eingespritzt. Dieser Zustand ist in Fig. 1c dargestellt. Besonders günstig ist eine (von Fig. 1a bis 1c abweichende) Ausbildung der Schleuseneinrichtung derart, daß die Nadel vollständig durch das Ventil hindurchgeht und in den Verbrennungsraum ragt, wenn die Probe eingespritzt wird.

Nach vollständigem Einbringen der Probenflüssigkeit in den Verbrennungsraum werden die oben zitierten Bewegungsabläufe in umgekehrter Reihenfolge wiederholt, so daß auch nach dem Einbringen der Probe der Verbrennungsraum zur Umgebung hin verschlossen bleibt. Die entleerte Injektions- bzw. Probenahmespritze 15 wird nach ihrer Herausführung aus der Schleuse 3 in dem Raumbereich zwischen dem Verbrennungsofen und einem (in den Fig. 1a bis 1c nicht dargestellten) Probenreservoir bis zur nächsten vorgesehenen Probenahme in Bereitschaft gehalten.

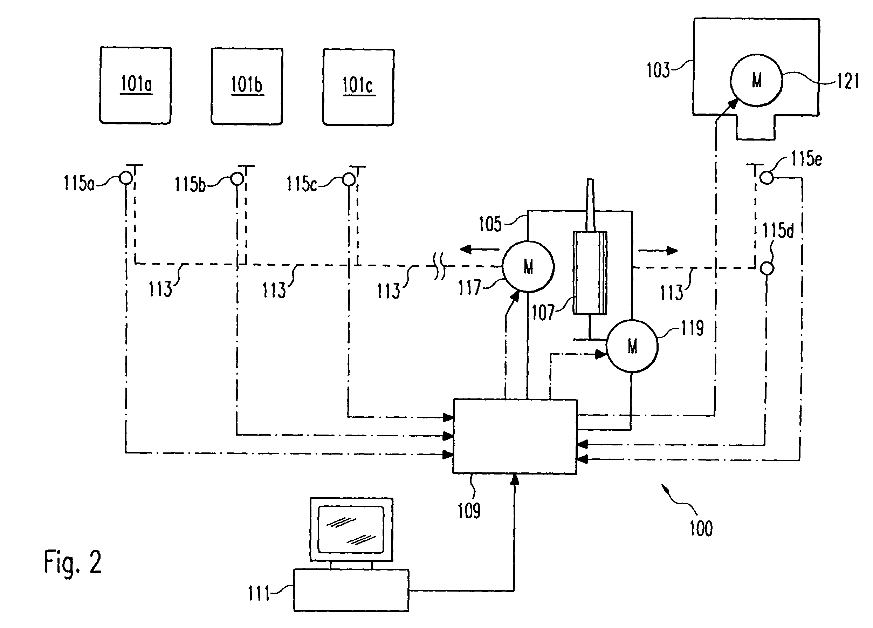

Fig. 2 zeigt eine Prinzipskizze einer Anordnung 100 zur Entnahme einer Abwasserprobe aus verschiedenen Behältern 101a bis 101c und zur Überführung der Probe in einen Verbrennungsofen 103. Die Beschickung des Verbrennungsofens 103 erfolgt mittels eines Beschickungsroboters 105, der eine Probenahmespritze 107 trägt und eine Koordinatensteuereinrichtung 109 aufweist, die mit einem PC 111 verbunden ist. Die möglichen Bewegungsbahnen des Beschickungsroboters 105 sind in der Figur als gestrichelte Linien gezeichnet und mit der Ziffer 113 bezeichnet und führen von den Behältern 101a bis 101c zum Verbrennungsofen 103.

Den zu den Behältern 101a bis 101c hin führenden Abschnitten der Bewegungsbahnen 113 sind Positionsfühler 115a bis 115c zugeordnet, und dem zum Verbrennungsofen 103 hin führenden Abschnitt der Bewegungsbahn sind zwei Positionsfühler 115d und 115e zugeordnet. Mittels der Positionsfühler wird die Position der Probenahmespritze 107 sowohl vor der Phase des Aufziehens der Spritze mit Abwasserproben aus einem der Behälter als auch in der Phase der Beschickung des Verbrennungsofens erfaßt. In der letzteren Phase sind - wie sich bereits aus den vorangehenden Ausführungen ergibt - zwei Positionserfassungsschritte erforderlich, weshalb an dem zum Verbrennungsofen 103 führenden Abschnitt der Bewegungsbahn 113 zwei Positionsfühler vorgesehen sind. Alle Positionsfühler 115a bis 115e sind Eingängen der Koordinatensteuereinrichtung 109 verbunden, und diese ist ausgangsseitig mit einem Antriebsmotor 117 des Beschickungsroboters, einem mit dem (nicht gesondert bezeichneten) Kolben der Probenahmespritze 107 verbundenen Spritzenbetätigungsmotor 119 sowie einem Ventilbetätigungsmotor 121 zur Betätigung des Kugelventils 13 (Fig. 1a bis 1c) des Verbrennungsofens verbunden.

Die Betriebsweise dieser Anordnung ist prinzipiell die folgende: Gemäß einem über den PC 111 eingegebenen Probenahmeprogramm wird in vorbestimmten Zeitabständen und vorbestimmter Reihenfolge mittels der Probenahmespritze 107 eine Abwasserprobe aus einem der Behälter 101a bis 101c entnommen. Hierzu wird der Beschickungsroboter 105 unter Steuerung durch die Koordinatensteuereinrichtung 109 in die entsprechende Probenahmeposition verfahren und dort durch den Spritzenbetätigungsmotor 119 die Probenahmespritze 107 aus dem ausgewählten Behälter aufgezogen. Anschließend fährt der Beschickungsroboter 105 längs der Bewegungsbahn 113 vor den Verbrennungsofen 103, wo der Positionsfühler 115d bei Erreichung der in Fig. 1b gezeigten Position - also nach dem ersten Vorschubschritt auf dem dem Verbrennungsofen 103 zugeordneten Abschnitt der Bewegungsbahn 113 - ein Signal an die Koordinatensteuereinrichtung 109 ausgibt, die den Beschickungsroboter 105 in dieser Position anhält. Anschließend gibt sie ein Steuersignal an den Ventilbetätigungsmotor 121 aus, der das Kugelventil 11 in die Öffnungsstellung dreht. Nachdem dies geschehen ist, leitet die Koordinatensteuereinrichtung 109 den zweiten Vorschubschritt ein, der die Probenahmespritze in die in Fig. 1c gezeigte Position bringt. Nachdem der dieser Position zugeordnete Positionsfühler 115e ein entsprechendes Signal ausgegeben hat, gibt die Koordinatensteuereinrichtung 109 ein Steuersignal an den Spritzenbetätigungsmotor 119 zum Entleeren der Probenahmespritze 107 in den Verbrennungsofen 103 aus. Nachdem die Spritze entleert ist, wird sie wieder so weit zurückgefahren, daß der Verbrennungsofen verschlossen werden kann, und dann in einem zweiten Schritt aus diesem herausgeführt und in eine Bereitschaftsstellung gebracht, in der sie bis zum nächsten Probenahmeschritt verbleibt.

Die Ausführung der Erfindung ist nicht auf das oben beschriebene Beispiel beschränkt, sondern auch in einer Vielzahl abgewandelter Ausführungen möglich. So kann die Beschickung des Verbrennungsofens auch halbautomatisch oder manuell erfolgen, und die Probe kann auch direkt aus einem von der zu untersuchenden Flüssigkeit durchströmten Fluidkanal entnommen werden. In einer Abwandlung der oben beschriebenen Anordnung weist die Probenahmespritze eine über einen Schlauch mit dem Spritzenkörper verbundene Nadel auf, und nur diese wird - bei stationärem Spritzenkörper - zwischen Probebehälter(n) und Verbrennungsofen verfahren.

Anstelle einer in Art einer Injektionsspritze ausgeführten Probenahmespritze kann auch eine anders ausgebildete Probenahmeeinrichtung vorgesehen sein, solange zum Zusammenwirken mit dem erfindungsgemäßen Verbrennungsofen geeignet ist.

Bei Einsatz von Schrittmotoren zum Antrieb der Verfahreinrichtung können die Positionsfühler oder zumindest ein Teil davon entfallen, da dann der Vorschub auch ohne Positionserfassung mit guter Präzision und Reproduzierbarkeit gesteuert werden kann. Gleichwohl können auch in diesem Fall Positionsfühler als Sicherheitsabschalter vorgesehen sein, mit denen Beschädigungen der Schleuseneinrichtung und/oder der Probenahmeeinrichtung vermieden werden.

### Bezugszeichenliste

- 1: Verbrennungsofen
- 3: Schleuseneinrichtung
- 5: Ofeninnenraum
- 7: erster äußerer Wandungsabschnitt
- 9: Führungsring
- 9a: zentrische Öffnung
- 11: zweiter äußerer Wandungsabschnitt
- 13: Kugelventil
- 13a: Fluidkanal
- 15: Probenahmespritze
- 15a: Nadel
- 100: Anordnung zur Entnahme einer Abwasserprobe
- 101a: Behälter
- 101b: Behälter
- 101c: Behälter
- 103: Verbrennungsofen
- 105: Beschickungsroboter
- 107: Probenahmespritze
- 109: Koordinatensteuereinrichtung
- 111: PC
- 113: Bewegungsbahn
- 115a: Positionsfühler
- 115b: Positionsfühler
- 115c: Positionsfühler
- 115d: Positionsfühler
- 115e: Positionsfühler
- 117: Antriebsmotor
- 119: Spritzenbetätigungsmotor
- 121: Ventilbetätigungsmotor
- A: gemeinsame Achse
- R: Ventilöffnungsrichtung
- S1: erster Vorschubschritt
- S2: zweiter Vorschubschritt

## Patentansprüche

1. Verbrennungsofen (1; 103) für die Verbrennung von flüssigen Proben zur Bestimmung von Inhaltsstoffen, insbesondere von Wasser- oder Abwasserproben zur Bestimmung des Kohlenstoffgehalts,
**gekennzeichnet durch**
eine Schleuseneinrichtung (3) zum Eintrag der Probe mittels einer Probenahmeeinrichtung (15; 107).

2. Verbrennungsofen nach Anspruch 1,
**dadurch gekennzeichnet,**
daß die Schleuseneinrichtung (3) eine von der Außenseite des Verbrennungsofens (1) zugängliche, durch Einführung der Probenahmeeinrichtung (15) dicht verschließbare Beschickungsöffnung (9a) und einen der Beschickungsöffnung zum Inneren des Verbrennungsofens hin nachgeordneten steuerbaren Verschluß (13) umfaßt.

3. Verbrennungsofen nach Anspruch 2,
**dadurch gekennzeichnet,**
die Beschickungsöffnung (9a) der Schleuseneinrichtung (3) als im Querschnitt kreisförmige, an den Außenquerschnitt eines Eingriffsabschnitts (15a) der Probenahmeeinrichtung (15) angepaßte Öffnung eines elastischen oder elastisch gelagerten Führungsrings (9) ausgebildet ist.

4. Verbrennungsofen nach Anspruch 2 oder 3,
**dadurch gekennzeichnet,**
daß der Verschluß (13) ein manuell oder in Abhängigkeit vom Verschlußzustand der Beschickungsöffnung (9a) automatisch gesteuertes Absperrorgan, insbesondere ein Absperrventil oder einen Absperrhahn oder Absperrschieber, aufweist.

5. Verbrennungsofen nach Anspruch 4,
**gekennzeichnet durch**
die Ausbildung des Absperrorgans (13) derart, daß sein Öffnungsquerschnitt an den Außenquerschnitt des Eingriffsabschnitts (15a) der Probenahmeeinrichtung (15) angepaßt ist, derart, daß diese mindestens abschnittsweise durch das Absperrorgan, insbesondere bis in den Verbrennungsraum (5) hinein, in den Verbrennungsofen (1) eingeschoben werden kann.

6. Anordnung (100) zur Entnahme einer flüssigen Probe aus einem Reservoir (101a bis 101c) zu deren Überführung in einen Verbrennungsofen (1; 103) nach einem der vorangehenden Ansprüche,
**gekennzeichnet durch**
eine Verfahreinrichtung (105) zum Verfahren mindestens eines Abschnitts (15a) der Probenahmeeinrichtung (107; 15) zwischen dem Reservoir und dem Verbrennungsofen und zu deren mindestens abschnittsweise Einführung in die Schleuseneinrichtung (3) des Verbrennungsofens, wobei die Verfahreinrichtung einen elektromotorischen Antrieb (117) und eine Koordinatensteuereinrichtung (109) aufweist.

7. Anordnung nach Anspruch 6,
**dadurch gekennzeichnet,**
daß die Schleuseneinrichtung (3) einen, insbesondere elektromotorischen, steuerbaren Antrieb (121) aufweist.

8. Anordnung nach Anspruch 7,
**dadurch gekennzeichnet,**
daß die Koordinatensteuereinrichtung (109) zur Einführung mindestens eines Abschnitts (15a) der Probenahmeeinrichtung (15; 107) in den Verbrennungsofen (1; 103) in einem ersten und einem zweiten Schritt (51, 52), insbesondere in ein und derselben Vorschubrichtung, und zur Ausgabe eines Öffnungssteuersignals an den Antrieb (121) der Schleuseneinrichtung (3) nach dem ersten Schritt ausgebildet ist.

9. Anordnung nach Anspruch 8,
**dadurch gekennzeichnet,**
daß der Antrieb mindestens einen Schrittmotor umfaßt.

10. Anordnung nach einem der Ansprüche 6 bis 9,
**dadurch gekennzeichnet,**
daß der Verfahreinrichtung (105) eine vorbestimmte Bewegungsbahn (113) zugeordnet ist, der in einem dem Verbrennungsofen (103) benachbarten Abschnitt mindestens einen Positionsfühler (115d, 115e) zur Erfassung der Position der Verfahreinrichtung (105) bzw. der Probenahmeeinrichtung (107) bezüglich des Verbrennungsofens aufweist.
